(19) Europäisches Patentamt European Patent Office Office européen des brevets

(11) **EP 4 657 453 A1**

(12) **EUROPEAN PATENT APPLICATION**

(43) Date of publication:
03.12.2025 Bulletin 2025/49

(21) Application number: 25178439.3

(22) Date of filing: 23.05.2025

(51) International Patent Classification (IPC):
**G16H 40/20** (2018.01)

(52) Cooperative Patent Classification (CPC):
**G16H 40/20**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **29.05.2024 US 202418677318**

(71) Applicant: **Optellum Limited**
**Oxford, Oxfordshire OX1 4EH (GB)**

(72) Inventors:
• ELLIS, Barnaby George
Oxford, OX1 4EH (GB)
• CHOMETON, Quentin Pierre
Oxford, OX1 4EH (GB)
• MONTILVA, Carlos Federico Arteta
Oxford, OX1 4EH (GB)
• POTESIL, Vaclav
Oxford, OX1 4EH (GB)

(74) Representative: **Optimus Patents Limited**
**Peak Hill House**
**Steventon**
**Basingstoke, Hampshire RG25 3AZ (GB)**

(54) **SYSTEM AND METHOD FOR AUTOMATION OF PATIENT DISCOVERY AND WORKFLOW DISTRIBUTION**

(57) A workflow optimisation system and method for at least one of: prioritisation of patients and distribution of patients to a specified treatment pathway is described. The system comprising: a database comprising patient medical data; a patient discovery circuit for receiving medical data from the database to transform the received medical data to retrieved patient data; a feature extraction circuit configured to process the retrieved patient data to produce structured data for each of the patients, and aggregate the structured data to a single vector for each patient, where the single vector summarises medical features for the patient; and a patient distribution circuit for receiving the single vector for each patient and determining, from the single vector for each patient, at least one of: a patient prioritisation list to prioritise patients for distribution, and a patient distribution list to distribute the patients to one or more distribution targets.

FIG 2

EP 4 657 453 A1

## Description

## Field of Invention

This invention relates to the field of medical informatics and the optimization of clinical workflows using automated systems.

## Background of Invention

Healthcare providers such as hospitals and primary care clinics store and process vast quantities of patient related data. These data can take a plurality of forms, for example: clinical notes, radiology reports, medical images (X-Ray; Computed Tomography (CT); Ultrasound (US)) and electronic health records (EHR). The information contained within the data is used to direct the healthcare provider toward the best course of action for a given patient for a given medical condition. The parsing of this information is a crucial but often a manual and laborious process. Extracting informative features from unstructured data is complex, and typically requires multiple experts to interpret the different data modalities and jointly determine the best course of action. Moreover, the administrative cost of organising patients into different care pathways is high and lead to delays in patient care.

Manual patient management systems are prone to human error and biases. Patients which have an incidental finding are particularly vulnerable to this. Incidental findings are those discovered opportunistically during imaging studies and other diagnostic tests ordered for a different clinical problem. A systematic review revealed that 23.6% of diagnostic tests lead to an incidental finding, rising to 31.3% for CT imaging studies [1]. These incidental findings often risk being lost to follow-up because the ordering physician typically focuses on the primary indication for the study, neglecting additional findings. As a result, there is frequently no designated system responsible for managing these incidental findings, leading to potential oversight and mismanagement. The lack of standardized follow-up protocols and the high volume of incidental findings also contribute to the challenge, making systematic tracking and management essential to avoid missed diagnoses and ensure appropriate care.

Automated tools which augment and streamline the patient management process are commercially available and used in clinical practice. Optellum's Virtual Nodule Clinic (VNC) [2] periodically searches incoming radiology reports to identify patients with at least one lung nodule, prompting the clinical team to decide the appropriate course of action. Similarly, Eon's Patient Management (EPM) software [3] uses NLP to identify actionable findings for several conditions such as incidental pulmonary nodules and liver lesions. Another solution, contextflow SEARCH Lung CT [4] enables a user to mark a region of interest (ROI) on a CT. The software subsequently uses the region of interest to query a CT image database for similar CTs. This can be used, for example, to find patients with similar nodules (ground-glass, spiculated, sub-pleural). There are no known commercially available products which combine text and image-based data in a patient retrieval task.

These solutions primarily focus on the 'discovery' component of the patient care pathway. Patient discovery systems are often configured to have high sensitivity to avoid catastrophically missing a positive case. This high recall inevitably leads to many cases which do not require further action. This overload of information can be problematic to a clinician tasked with organising retrieved patients. There are tools which can automatically extract features from data such as radiology reports and CT images. The features typically describe a combination of patient attributes such as age, sex and family history; and attributes describing medical entities associated with a patient. In this context, a medical entity is a distinct finding within the data, such as a pulmonary nodule in a CT scan. For example, Optellum's VNC enables a pulmonologist to select nodules and evaluate the risk using the Lung Cancer Prediction (LCP) module of the software. LCP is a computer aided diagnosis (CADx) device which uses artificial intelligence to predict the likelihood of malignancy for a given nodule. The pulmonologist is then able to prioritise patients according to this risk.

Resource management in healthcare requires careful patient allocation to workflows with adequate capacity. For instance, a facility may have multiple pulmonologists, nurse practitioners and navigators, each able to monitor lung nodule patients. Assigning patients involves administrative costs and depends on the subjective assessment of the patient's condition and available resources. Efficient distribution is crucial to avoid overloading any single provider and to ensure timely, quality and cost-efficient care. This requires a balanced approach, integrating objective data on provider capacity and patient needs with the subjective judgment of healthcare professionals.

An effective automated patient management system would ensure patients do not fall through administrative cracks, whilst ensuring resource constrained healthcare service providers optimally follow up the most appropriate patients in a suitable order.

## References

[1] Lumbreras, B., Donat, L., Herna, I., & Hernandez, M. (n.d.). Incidental findings in imaging diagnostic tests: a systematic review. https://doi.org/10.1259/bjr/98067945

[2] https://optellum.com/products-and-solutions/tracking-and-management/

[3] https://eonhealth.com/

[4] Röhrich, S., Heidinger, B. H., Prayer, F., Weber, M., Krenn, M., Zhang, R., Sufana, J., Scheithe, J., Kanbur, I., Korajac, A., Pötsch, N., Raudner, M., Al-Mukhtar, A., Fueger, B. J., Milos, R.-I., Scharitzer, M., Langs, G., & Prosch, H. (n.d.). Impact of a content-based image retrieval system on the interpretation of chest CTs of patients with diffuse parenchymal lung disease. https://doi.org/10.1007/s00330-022-08973-3/Published

## Summary of the Invention

According to an example there s provided a workflow optimisation system to perform at least one of: prioritisation of patients for a specific treatment pathway; distribution of patients to a specified treatment pathway; the system comprising: a database comprising medical data for one or more patients; a patient discovery circuit for receiving medical data for one or more patients from the database to transform the received medical data to retrieved patient data; a feature extraction circuit configured to process the retrieved patient data to produce structured data for each of the one or more patients, and aggregate the structured data to a single vector for each patient, where the single vector summarises medical features for the patient; and a patient distribution circuit for receiving the single vector for each patient and determining, from the single vector for each patient, at least one of: a patient prioritisation list to prioritise patients for distribution, and a patient distribution list to distribute the patients to one or more distribution targets.

Preferably, the medical data comprises at least one of: medical imaging data, structured data, unstructured data.

In a preferred example, the patient prioritisation list and the patient distribution list can be reviewed and/or edited by a user of the system.

Further preferably, the medical data is retrieved from the database in response to one of more natural language queries.

In an example, the patient discovery circuit comprises a data transformation model to transform the received medical data, wherein the data is transformed by one or more of: -restructuring or reformatting the data, removing patient identifiable information, removing any other unnecessary data, producing data summaries, encoding raw data such medical images to facilitate their parsing, and a patient retrieval model, wherein the patient retrieval model analyses the transformed data.

Preferably, the patient retrieval model analyses the transformed data to determine the presence of at least one of: a predefined regular expression string (regex); a match to a predefined database query.

Further preferably, the data transformation model comprises an encoder for each of the medical imaging data, the structured data and the unstructured data, and each encoder outputs a vector for each of the medical imaging data, the structured data and the unstructured data, wherein the output vectors are input to a embedding merger to be fused to generate the single output vector for a patient.

In a preferred example, the output vectors for all patients are concatenated to generate a patient matrix, $X_p$. Futher preferably, the patient matrix $X_p$, is provided as an input to the data retrieval model, and a natural language query is input to a query encoder in the data retrieval model, and an output vector from the query encoder is provided to the data retrieval model, and is combined with the patient matrix to generate a relevance score, $r_q$, for each patient, which indicates how relevant is each patient in $X_p$ to the natural language query.

Preferably, the relevance score is generated using a similarity metric between the patient features and the encoded natural language query.

Further preferably, the retrieval model is configured to select one or more patients based on a comparison of the relevance score with a predetermined threshold.

In a preferred example, one or more of the encoders is a neural network.

Preferably, the feature extraction circuit comprises a feature extraction model to extract one or more feature vectors from the retrieved patient data and an aggregation model, that receives the one or more feature vectors, and produces an output of one aggregated vector per patient. Further preferably the aggregated vectors for each patient are concatenated to produce a structured patient database.

In a preferred example, the feature extraction model comprises the detection and characterization of a medical entity in the medical data, and outputs a feature vector comprising the entity location, detection confidence parameter, and entity characterization information for medical imaging data from one or more patients. Further preferably, the medical entity is a nodule, and the feature vector can be used to determine one or more of nodule malignancy risk, nodule size, nodule attenuation, and other clinical parameters for the nodule for one or more of the patients.

Preferably, the patient distribution circuit comprises a distribution model that receives information from the structured patient database and a target state encoder that also provides real-time information about the distribution targets as input to the distribution model, wherein the distribution model produces an output indicating a distribution of patients to distribution targets according to patient and distribution target requirements.

Further preferably, the distribution model is a static model, where the state of the distribution targets is fixed in time. Alternatively, the distribution model is a dynamic model, where the target state encoder generates a target state matrix, $X_T$, where $X_T$ will vary as a function of time.

In an example, there is provided a workflow optimization method for the prioritisation and distribution of patients to a specified treatment pathway comprising: receiving and storing medical data for one more patients in a patient database; receiving medical data for one or more patients from the database at a patient discovery circuit to transform the received medical data to retrieved patient data; processing the retrieved patient data in a feature extraction circuit to produce structured data for each of the one of more patients, and aggregating the structured data to a single vector for each patient, where the single vector summarises medical features for the patient; and receiving the single vector for each patient at a patient distribution circuit and determining at least one of: a patient prioritisation list and a patient distribution list to prioritize and distribute the patients to one or more distribution targets.

### Brief Description of the Figures

Further details, aspects and embodiments of the invention will be described, by way of example only, with reference to the drawings. In the drawings, like reference numbers are used to identify like or functionally similar elements. Elements in the figures are illustrated for simplicity and clarity and have not necessarily been drawn to scale.

Figure 1: illustrates a general configuration of the patient workflow optimisation system according to an embodiment of the invention;

Figure 2: illustrates a general configuration of the patient discovery circuit according to an embodiment of the invention;

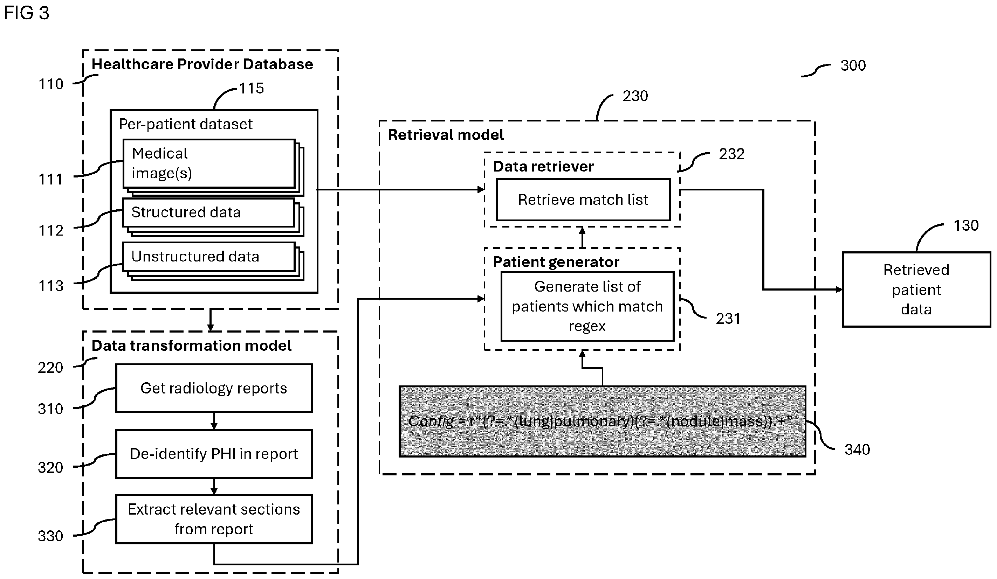

Figure 3: shows an example configuration of the patient discovery circuit which searches CT radiology reports for pulmonary nodules using regular expressions according to an embodiment of the invention;

Figure 4a: shows an example configuration of the data transformation model which encodes multimodal patient data to a shared embedding space according to an embodiment of the invention.

Figure 4b: shows an example configuration of the patient retrieval model which parses a natural language query and uses it to retrieve patients from the shared embedding space according to an embodiment of the invention;

Figure 5: illustrates a general configuration of the feature extraction circuit according to an embodiment of the invention;

Figure 6a: illustrates an example configuration of the feature extraction model which detects, scores and measures pulmonary nodules according to an embodiment of the invention;

Figure 6b: illustrates an example configuration of the aggregation model according to an embodiment of the invention;

Figure 7a: illustrates a configuration of the patient distribution circuit according to an embodiment of the invention;

Figure 7b: illustrates a configuration of the patient distribution circuit with user intervention according to an embodiment of the invention;

Figure 8: illustrates an example configuration of the patient distribution circuit which distributes patients to the either a nodule clinic or a tumour board according to an embodiment of the invention;

Figure 9: illustrates an example configuration of the patient distribution circuit which combines patient attributes and target attributes to distribute patients according to an embodiment of the invention.

### Detailed Description

### Overview of the invention

This invention (100) describes a workflow optimisation method and system to assist in the discovery, pre-prioritization and distribution of patients for a care pathway within a healthcare system. This invention targets the optimisation of a workflow comprising three distinct phases: (i) patient discovery, (ii) feature extraction and (iii) patient distribution.

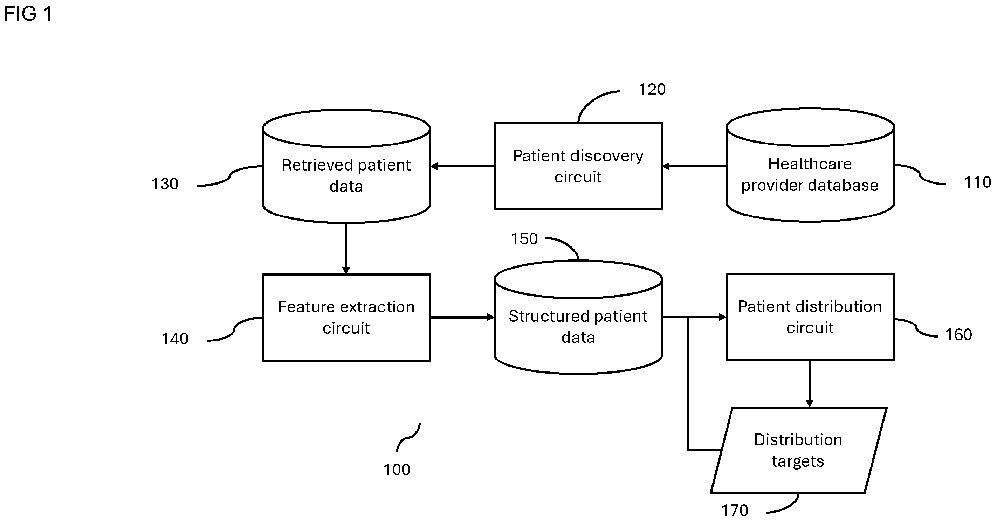

The system, as shown in FIGURE1, includes: a patient discovery circuit (120), which parses the database of a healthcare provider (e.g. hospital, primary care centre) (110) hosted on-premise or remotely to retrieve the raw clinical data of relevant patients (130); a feature extraction circuit (140), which extracts features from the retrieved patient data (130) to output a structured array of patient data (150); and a patient distribution circuit (160) which uses the structured patient data (150) to distribute each patient to an appropriate distribution target (170) according to an optimisation method. The patient distribution circuit may also use as input the current state of the distribution targets (170). As described, a workflow optimisation system for at least one of: prioritisation of patients and distribution of patients to a specified treatment pathway comprises: a database comprising medical data for one more patients; a patient discovery circuit for receiving medical data for one or more patients from the database to transform the received medical data to retrieved patient data; a feature extraction circuit configured to process the retrieved patient data to produce structured data for each of the one of more patients, and aggregate the structured data to a single vector for each patient, where the single vector summarises medical features for the patient; and a patient distribution circuit for receiving the single vector for each patient and determining, from the single vector for each patient, at least one of: a patient prioritisation list to prioritise patients for distribution, and a patient distribution list to distribute the patients to one or more distribution targets.

The healthcare provider database (110) may contain a plurality of different data modalities associated with a patient. These include but are not limited to: medical images such as computerized-tomography (CT) scans, Positron Emission Tomography (PET-CT), X-rays, ultrasound (US), and magnetic resonance imaging (MRI) scans; structured data such as clinical data (age, ethnicity, gender, family history, symptoms); and unstructured data such as narrative radiology reports and clinical notes. In an example, there is no limit on the time period that the medical data may cover. Preferably, the data stored in the database may comprise one or more of medical imaging data, structured data, unstructured data for one or more of the patients.

With the automatic discovery, organisation and distribution of patients, healthcare providers can invest more resources into patient care whilst maintaining confidence that the patients receive timely and appropriate referrals. The workflow can also be implemented in a clinical research context, for example, in which a clinical research organisation wants a curated list of patients meeting the criteria for a clinical trial.

The various elements of the patient workflow optimisation system (100) are detailed next.

**Patient discovery circuit**

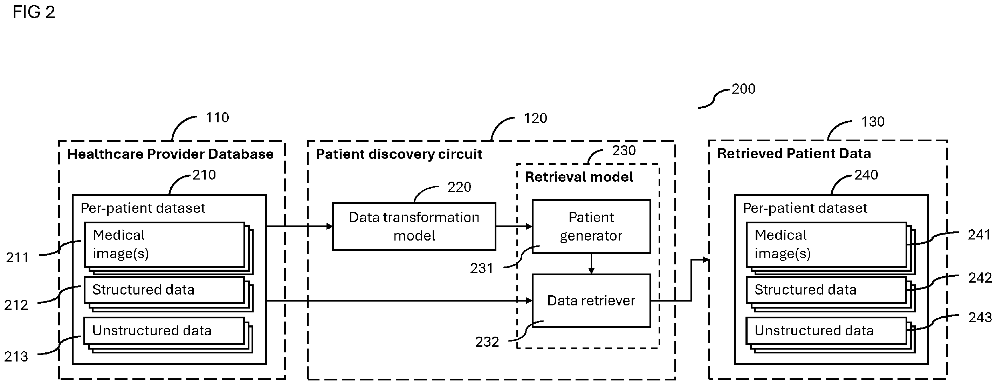

The patient discovery circuit (120) constitutes the first part of the patient workflow optimisation system (100). The general configuration (200) of the patient discovery circuit (120), including its input and output, is shown in FIGURE 2. The circuit takes as input the healthcare provider database (110) containing multimodal datasets (210) associated with one or more patients. The data can be categorised into: medical images (211) such as CT scans, MRI images, PET scans; structured data (212) such as patient demographic data and results from clinical exams such as blood tests results; and unstructured data (213) such as narrative radiology reports and clinical notes. At least part of the multimodal data first passes through the data transformation model (220) which is configured to transform and/or sample the data into the form required by the downstream patient retrieval model (230). For example, the data transformation model (220) may perform one or more of: restructuring or reformatting the data, removing patient identifiable information or any other unnecessary data, producing data summaries, and encoding raw data such medical images to facilitate their parsing. These tasks may be done in any order, and in any combination, according to the desired results.

The transformed data then passes to the patient retrieval model (230), which preferably contains a patient generator (231) and a data retriever (232). The patient generator (231) preferably generates one or more subsets of patient indices which are to be retrieved, for example, unique patient identifiers and/or location of patient data within the hospital database (110). The data retriever (232) uses the generated indices to retrieve the relevant patient data from the healthcare provider database (110). The output of the retrieval model (230), which is directly output by the patient discovery circuit (120), is all the available data associated with the subset of patients determined by the patient generator (231) and retrieved by the data retriever (232). The data retriever 232 then produces retrieved patient data 240, with a per patient dataset comprising retrieved data 241 corresponding to the medical images 211, retrieved data 242 corresponding to the structured data 212, and retrieved data 243 corresponding to the unstructured data 243.

The patient retrieval model (230) may also take additional inputs which define the criteria of the patient generation model. These inputs can take multiple forms, such as database queries, regular expression strings or natural language queries. In embodiments of the patient retrieval model which do not take additional inputs, the patient generator criteria are typically constant and built into the patient generator. For example, the patient generator may always use the same regular expression pattern to search radiology reports, so there is no requirement to pass inputs that modify the search criteria.

The configuration of the patient discovery circuit (120) can take various forms depending on the task, multiple examples are described next.

*Search radiology reports for patients with a reported entity*

In this embodiment (300) of the patient discovery circuit (120), details for patients with a medical report which contains keyword(s) indicating the presence of a particular entity are retrieved for the medical database. Preferably, the report is a radiology report and the report may indicate the presence of a medical entity. In this context, an entity is defined as a specific anatomical structure, pathological condition, or diagnostic finding that can identified in the text of a radiology report. In a preferred example the medical entity may be a lung nodule.

Multimodal datasets from patients (115) in a healthcare provider database (110) are first passed to the data transformation model (220), which is configured to first extract the most recent radiology report for each patient (310). Patients who do not have a radiology report are discarded from the set of patients at this point. The data transformation model then removes any identifiable personal health information (PHI) from each of the extracted radiology report (320) and removes any sections of the radiology report which do not describe the present radiological examination (330), such as patient/family history. In this way, only the necessary data is passed to the retrieval model for further analysis.

The data as transformed by the transformation model (220) is then passed to the retrieval model 230, and preferably passed to the patient generator (231) within the retrieval model 230, which is configured to find reports which contain text patterns specified in the parameters. In this embodiment, the text patterns are represented using regular expression (regex) strings (340), which are configured to assert the presence of the entity of interest. Preferably, the patient discovery circuit comprises a data transformation model to transform the received medical data, and a patient retrieval model, wherein the patient retrieval model analyses the transformed data to determine the presence of a predefined regular expression string (regex). In an example of this embodiment, the given entity is a pulmonary nodule, which are found using the following regex string:

$$r"(?=.*(lung|pulmonary))(?=.*(nodule)|(lesion)|(opacity)).+"$$

This regex matches lines of the text where either 'lung' or 'pulmonary' and either 'nodule', 'lesion' or 'opacity' occur. Any patient for which a match occurs is added to the subset to be used to retrieve the raw data using the data retriever (232), within the retrieval model 230, which outputs this raw data to the retrieved patient data storage (130).

*Multi-modal search based on natural language queries*

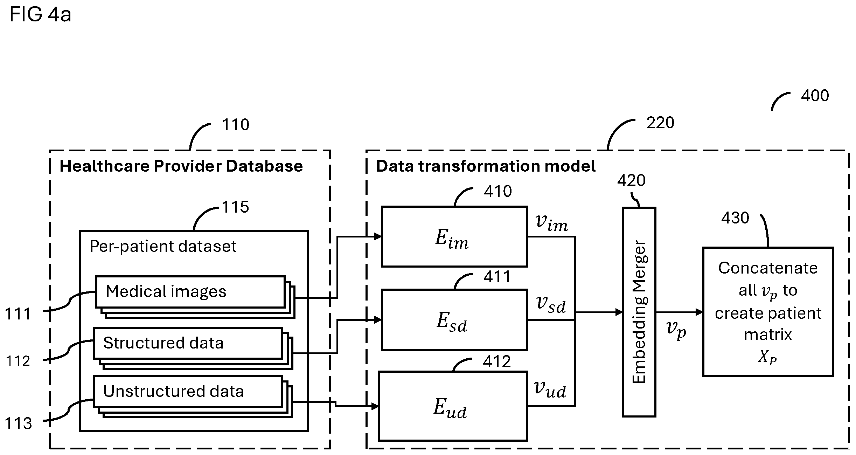

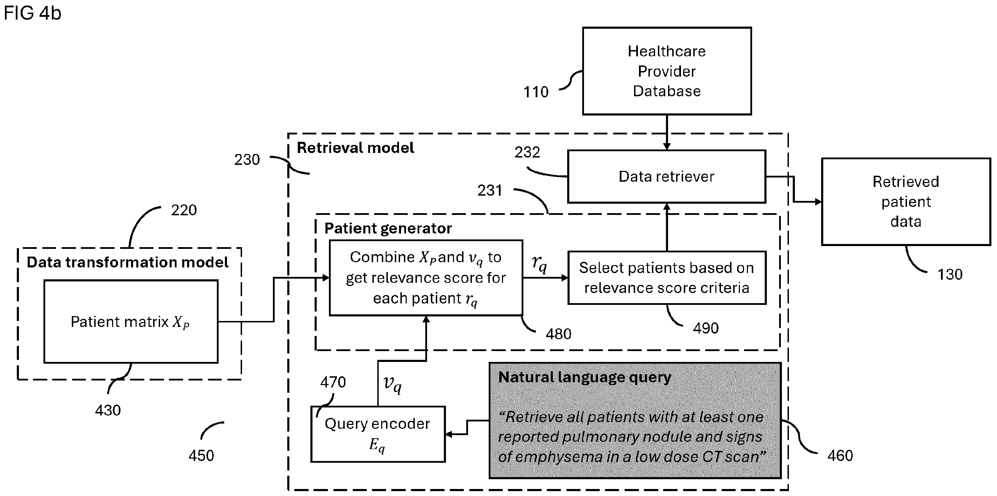

In this embodiment of the patient discovery circuit (120), natural language queries entered by a user of the workflow optimisation system (100) are parsed and used to retrieve patients from a database which are relevant to the query. A depiction of this embodiment is shown in FIGURE 4a and 4b. FIGURE 4a is an example configuration (400) of the data transformation model (220), and FIGURE4b (450) is an example configuration of the patient retrieval model (230). Preferably, some or all of the medical data is retrieved from the database in response to one of more natural language queries, that may be input by a system user.

Performing a multi-modal search from a natural language query first requires embedding one or more of the different data modalities into a shared latent space. In this embodiment, the data transformation model (220) takes as input a patient database containing medical images (111), structured clinical data (112) and unstructured clinical data (113) such as narrative radiology reports. Preferably, the data transformation model comprises an encoder for each of the medical imaging data, the structured data and the unstructured data, and each encoder outputs a vector for each of the medical imaging data, the structured data and the unstructured data, wherein the output vectors are input to a embedding merger to be fused to generate the single output vector for a patient. Each of the $k$ data modalities is then passed to an encoder $E_k$ which projects the respective data modality onto a vector $v_k \in \mathbb{R}^{d_k}$, where $d_k$ is the number of dimensions in the vector for data modality $k$. In certain configurations of this embodiment, at least one of the medical image encoder $E_{im}$ (410), structured data encoder $E_{sd}$ (411) and unstructured data encoder $E_{ud}$ (412) is a neural networks which has been jointly trained to maximise the similarity between $v_{im}$, $v_{sd}$ and $v_{ud}$ for a given patient. Preferably, all of the encoders will be neural networks. For example, a database of retrospectively collected datasets containing examples of each data modality for a large set of patients is assembled, such that the data in each of the modalities have corresponding semantic information. For instance, the data for a patient can consist of a medical image, a radiological report of the medical image, and electronic health record data for the patient. Preferably, all patients in the database will have this information. The various modalities are then processed by the corresponding neural networks, producing the vectors $v_{im}$, $v_{sd}$ and $v_{ud}$ for a given patient, relating to the image data, the structured data and the unstructured data respectively. The distance between the vectors is then measured, preferably using a distance function such an L2 distance, and the parameters of the various neural networks are then updated as to reduce the distance between the embedding vectors, for instance, using the back-propagation algorithm. The process is repeated iteratively for all patients in the database until a convergence criterion is reached. Once convergence is reached, the various neural networks obtained are used to generate the various modality vectors $v_{im}$, $v_{sd}$ and $v_{ud}$ for any new patient being processed by the data transformation model (220).

In an example of this embodiment, the separate embeddings $v_{im}$, $v_{sd}$ and $v_{ud}$ are fused in an embedding merger (420), which outputs a single vector $v_p$ for a given patient. In some configurations of this embodiment, the embedding merger calculates a weighted sum of the individual vectors $v_{im}$, $v_{sd}$ and $v_{ud}$. The vectors $v_{pi}$ for all $i = [1, N_P]$, where $N_P$ is the number of patients, are concatenated to produce a patient matrix $X_P$, with $N_P$ rows and $d_p$ columns, where $d_p$ is the number of dimensions in $v_p$.

An example of the patient retrieval model for this embodiment (450) is shown in FIGURE 4b. The user inputs a query (460), preferably a natural language query, which is encoded by the query encoder $E_q$ (470), outputting a vector $v_q$ which has the same features as the patient matrix (430). In an example, the query may be "retrieve all patients with at least one reported pulmonary nodule and signs of emphysema in a low dose CT scan". In some example embodiments, $E_q$ is jointly trained with one or more of the data encoders $E_{im}$, $E_{sd}$, $E_{ud}$ as part of the end-to-end model optimisation. For instance, $E_q$ is built as an additional neural network on top of the encoder for unstructured data $E_{ud}$ in order to produce an encoding vector $v_q$ that has dimensionality $d_p$, and the parameters of $E_q$ are determined in the same iterative way as the multi-modality encoders. In another example embodiment, the training of $E_q$ is isolated from the training of the data encoders, such that the input to $E_q$ are the vectors $v_p$ produced by trained data encoders with fixed parameters. At each step in the training loop, a query associated with the patient encoded by the data encoders is encoded by $E_q$. The patient query is randomly sampled from a list of relevant queries for a given patient. For example, given a patient with a CT showing signs of lung cancer, a family history of lung cancer and multiple reported pulmonary nodules, a list of relevant queries may contain the following:

*"Retrieve patients showing signs of lung cancer"*
*"Get suspicious patients with multiple reported pulmonary nodules"*
*"Find a set of patients who have family history of lung cancer and at least one suspicious pulmonary nodule "*

The query vector $v_q$ is compared with the patient vector $v_p$ using a distance metric such as L2 distance, and the parameters of the various neural networks are updated as to reduce the distance between $v_q$ and $v_p$, for instance, using the back-propagation algorithm. The process is repeated iteratively for all patients in the database until a convergence criterion is reached. Once convergence is reached, $E_q$ is used to encode a given natural language query. For example, natural language queries can take the form of the following examples:

*"Find patients with lung nodules larger than 6mm and without a known history of cancer"*
*"Retrieve cases with lung masses and ages in the range of 20 to 70 years"*
*"Find patients undergoing cancer surveillance for one of the following cancers: breast, colorectal or head and neck, but not lung cancer"*

As noted in these examples, natural language queries naturally account for statements that include logical operators and comparators. For example, the negation, intersection, and union of queries (e.g. "not", "and", "or") or comparators such as larger/greater, equal or smaller/less than given values.

In an example of this embodiment, processing a natural language query (460) in order to perform a multi-modality search requires passing the encoded query $v_q$, as produced by $E_q$ (470) to the patient generator (231), which also takes as input the patient matrix $X_P$ (430). The patient generator (231) then combines $v_q$ with $X_P$ to generate a relevancy vector $r_q$ (480), containing the relevancy of the query to each of the $N_P$ patients. Preferably, the relevance score is generated using a similarity metric between the patient features and the encoded natural language query, and one or more patients will be selected after comparison of the relevance score with a predetermined threshold. In an embodiment, the one or more patients will be selected if the relevancy score exceeds the predetermined threshold, or alternatively the one or more patients will be selected if the relevancy score is below or equal to the predetermined threshold. In an example, a predefined number of patients, N, may be selected, based on the relevance score for each patient, without reference to a threshold and merely based on the relevance score alone, so that the top N most relevant cases are selected. In some example configurations, the relevancy score can be obtained using a similarity metric such as cosine similarity, which is calculated as follows:

$$\cos \theta = \frac{v_q \cdot X_P^T}{||v_q|| \; ||X_P||} = r_q \qquad (1)$$

The user can configure the patient discovery model to retrieve only patients with a relevancy above a given threshold. Alternatively, users can sort the list by similarity $r_q$ and select the top N patients to retrieve, where N is decided according to an expectation of the workload that the users may be able to process. The indices of the selected patients are output by the patient generator (490), which are used by the data retriever (232) to retrieve the raw data from the selected patients from the database 110. The selected raw data is output to the retrieved patient data storage (130).

In an example of this embodiment, there is a narrative radiology report and CT image for each patient in the healthcare provider database (110). The image encoder $E_{im}$ (410) projects the CT image onto a two-dimensional vector $v_{im}$, where the meaning of the features are as follows:

$$v_{im}(0) \in \{-1, 1\} \rightarrow \text{Resolution of the image, where 1 is highest resolution and -1 is lowest resolution}$$

$$im(1) \in \{-1, 1\} \rightarrow \text{Contrast level, where 1 is high contrast and -1 is low contrast.}$$

The encoder for unstructured data $E_{ud}$ (412) encodes the radiology report onto a four-dimensional vector $v_{ud}$, where the meaning of the features are as follows:

$$v_{ud}(0) \in \{-1, 1\} \rightarrow \text{Report contains a new pulmonary nodule.}$$

$$v_{ud}(1) \in \{-1, 1\} \rightarrow \text{The largest pulmonary nodule in the report is larger than 15 mm}$$

$$v_{ud}(2) \in \{-1, 1\} \rightarrow \text{There is reported family history of cancer}$$

$$v_{ud}(3) \in \{-1, 1\} \rightarrow \text{High contrast specified in the report}$$

Merging the two vectors is achieved by concatenating $v_{im}$ and $v_{ud}$ to get a six-dimensional vector $v_P$ for each patient, where the meaning of the features is the same as in $v_{im}$ and $v_{ud}$.

For example, consider a case where there are three patients in the database 110:

Patient 1: Has a thick-slice CT with low contrast. No reported pulmonary nodules and no mention of family history of cancer or contrast level in the report.

$$v_{im} = (-0.8, -1); \quad v_{ud} = (-1, -1, -1, -1)$$

Patient 2: Has a thin-slice CT with high contrast. There are pulmonary nodules where the largest is 10 mm. There is family history of lung cancer and high contrast is mentioned in the technique section of the report.

$$v_{im} = (0.8, 0.9) \quad v_{ud} = (1, -0.5, 1, 1)$$

The merging of the vectors for patients 1 and 2 forms the following patient matrix $X_P$:

$$X_P = \begin{bmatrix} -0.8 & -1 & -1 & -1 & -1 & -1 \\ 0.8 & 0.9 & 1 & -0.5 & 1 & 1 \end{bmatrix}$$

The following natural language query:

"Retrieve patients with a family history of cancer who have a reported pulmonary nodule", is encoded using the query encoder, producing $v_q$:

$$v_q = (0, 0, 1, 0, 1, 0)$$

The cosine similarity between $v_q$ and $X_P$ is computed using equation 1, which yields the relevancy vector r:

$$r_q = (-0.56, 0.65),$$

which indicates that patient 2 is the most relevant to the search. Given the range of $\cos\theta$ is well defined ($-1 < \cos\theta < 1$), an appropriate similarity threshold can be configured by the user.

In this example, for a similarity threshold of 0, the index of patient 2 is given by the patient generator (231) to the data retriever (232), which then fetches all data of patient 2 into the retrieved patient data storage (130).

**Feature Extraction circuit**

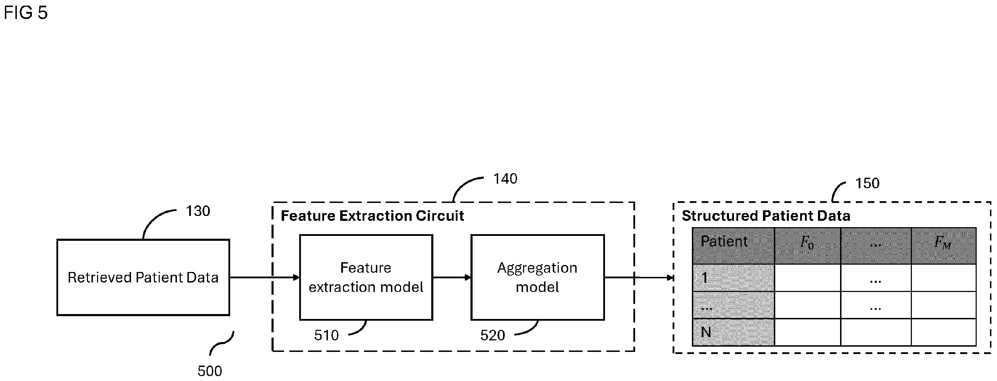

The feature extraction circuit (140) constitutes the second part of the patient workflow optimisation system (100), and follows the patient discovery circuit (120). An example general configuration (500) of the feature extraction circuit (140) is shown in FIGURE 5. The feature extraction circuit passes the retrieved patient data (130) through a feature extraction model (510) and an aggregation model (520) to output patient data (150), preferably as a structured array of data, each with a set of features required for the patient distribution circuit (160). Preferably, the feature extraction circuit 140 comprises an feature extraction 510 to extract one or more feature vectors from the retrieved patient data 130 and an aggregation model 520, that receives the one or more feature vectors, and produces an output of one aggregated vector per patient.

The feature extraction model (510) preferably performs the following steps: takes as input the retrieved raw patient data (130) from the patient discovery circuit (120); for one or more patients, extracts $N_f \in \mathbb{Z}_{\geq 0}$ feature vectors $v_{fj}$ for $j = [1, N_f]$, per patient from the retrieved patient data; and passes the vectors $v_{fj}$ per patient to the aggregation model (520). The feature extraction model (510) can be any model or combination of models which extracts feature vectors from raw patient data. In some examples of the feature extraction circuit (140), the feature extraction model (510) extracts features from imaging data using one or more sub-models that can detect, measure and characterize entities of interest. For instance, the feature extraction model (510) can detect an entity and extract their location in the medical image, alongside any characteristic relevant for deciding on the clinical pathway for the patient. For example, if the entity is a lung nodule, the feature extraction model (510) may extract the lung nodule location, size, attenuation, likelihood of malignancy, and other characterizations of a potential malignancy such as aggressiveness and predicted histological type. In another example, the feature extraction model extracts features from narrative reports such as measurements provided by a reporting radiologist in reference to entities of interest such as suspicious lesions present in a medical imaging exam, or symptoms presented by a patient when visiting a clinician as described in the clinical notes. In a further example, the feature extraction model (510) extracts features from structured data such as the clinical history of a patient. For instance, history of disease, smoking status, history of exams and interventions, and the results of clinical tests. The various configurations of feature extraction model (510) also allow to report the absence of the features that they are configured to extract. For instance, when configured to find the history of cancer of a given patient, the feature extraction model (510) can extract that not history of cancer is reported.

The aggregation model (520) takes as input the $N_f$ feature vectors $v_{fj}$ per patient output by the feature extraction model (510), and applies a function which reduces the number of feature vectors to one aggregated feature vector $v_a$ per patient. The number of features in $v_a$, $M \in \mathbb{Z}_{\geq 0}$, may be different to the number of features in each of $v_{fj}$. For example, the aggregation model may determine the $v_{fj}$ with the maximum value of a given feature, and output this as $v_a$, which has the same number of features as $v_{fj}$. In more complex examples, the aggregation model (520) may be a function of a plurality of features in $v_{fj}$. Preferably, the aggregated vectors for each patient are concatenated to produce a structured patient database. The vector $v_a$ from each patient are concatenated to form the structured patient dataset (150), which is passed to the patient distribution circuit (160). The number of feature vectors for a given patient after either the feature extraction model or the aggregation model may reduce to zero. In these circumstances, the patient is discarded.

Various example embodiments of the feature extraction circuit (140) are described below. The example embodiments are related to the extraction of patient level features associated with pulmonary nodules, which are relevant to a workflow optimisation system (100) configured for the scenario of discovery and distribution of patients under increased risk of lung cancer. Preferably, the feature extraction model comprises the detection and characterization of a medical entity, and outputs a feature vector comprising the entity location, detection confidence parameter, and entity characterization information for medical imaging data from one or more patients. In an example embodiment, the feature extraction model comprises the detection and characterization of a medical entity, and outputs a feature vector comprising the entity location, detection confidence parameter, and entity characterization information for medical imaging data from one or more patients.

*Extraction of size of the largest nodule in narrative radiology reports*

In this embodiment, the feature extraction model parses narrative radiology reports containing details of nodules, where the reports are retrieved in the discovery circuit (120). The feature extraction model (510) extracts the measurement(s) linked to pulmonary nodules from the radiology report. In this embodiment, the feature extraction model is a regular expression string which matches measurements. The regular expression string is configured to match one-, two- or three-dimensional measurements. The output of the feature extraction model is at least one feature vector for each patient, where the feature vector represents a single measurement, and has the following features:

x measurement (mm)
y measurement (mm)
z measurement (mm)

For example, the sentence: "There is a 10 x 5 x 4 mm nodule and a 2 cm nodule" will yield two feature vectors when passed to the feature extraction model:

$$v_{f1} = [10, 5, 4]$$

$$v_{f2} = [20, null, null]$$

The aggregation model (520) takes the two feature vectors $v_{f1}$, $v_{f2}$, determines the maximum measurement for each feature vector, then calculates the maximum of this measurement over the two feature vectors, which in this example is 20. The output of the aggregation model (520) in this embodiment of the feature extraction circuit (140) is a feature vector, where the sole feature is the maximum size, which for the patient in this example is 20mm. This process is repeated for all patients that have been retrieved in the patient discovery circuit (120), and the $v_a$ from each patient are concatenated to form a structured patient dataset (150).

*Extraction of lung nodule features from CT images.*

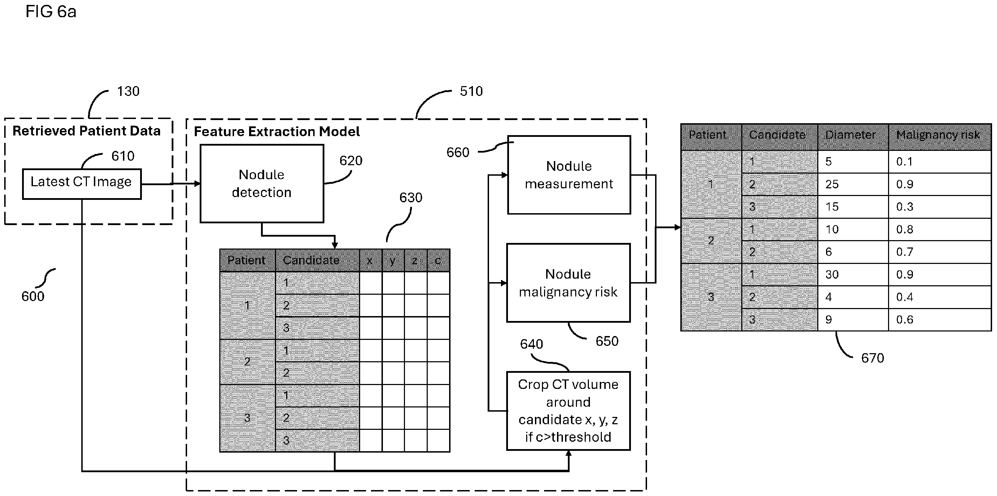

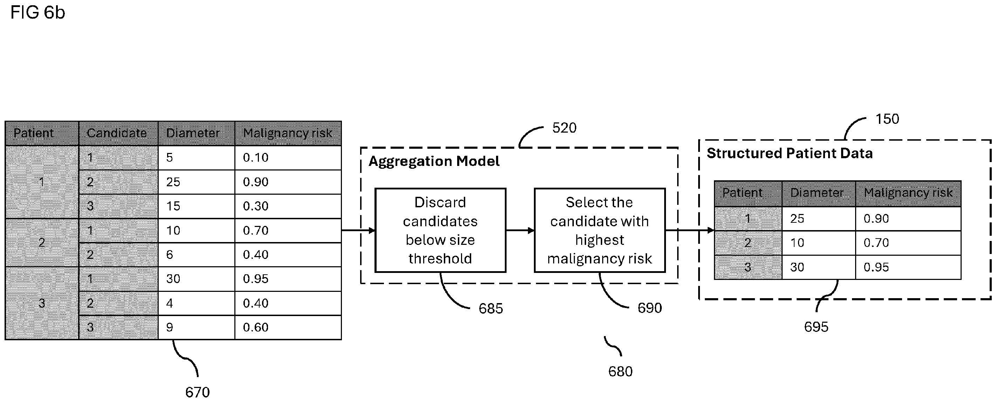

In this embodiment of the feature extraction circuit (140), depicted in FIGURE 6a and 6b, the feature extraction circuit is configured to detect and characterize lung nodules from CT images (600) and filter and aggregate the results (680) into structured patient data. The feature extraction model (510) takes as input the latest medical scan image from the patient data retrieved (130) in the patient discovery circuit (120), preferably the medical scan image is a CT scan, and performs the following steps:
Nodule detection (620): takes as input the most recent CT scan (610) with a reported pulmonary nodule and yields a feature vector $v_d$ for every nodule detected, which are output in the form of a list of nodule detections and corresponding feature vectors (630). In a preferred example, the feature vector $v_d$ contains four features: *x* location; *y* location, *z* location and detection confidence, which indicates the likelihood of the detection corresponding to a lung nodule.
CT cropping (640): for all detections which have a detection confidence above a pre-determined threshold, crop a sub-volume centred at the location specified by the *x, y, z* location components of $v_d$.
Nodule malignancy risk (650): for each detected nodule, takes as input the cropped CT from the CT cropping module (640), and yields a feature vector $v_r$ for every crop. The feature vector contains a single feature representing a malignancy score such as the probability of malignancy for the nodule.
Nodule measurement (660): for each detected nodule, takes as input the cropped CT from the CT cropping module (640), and yields a feature vector $v_m$ for every crop. The feature vector contains a single feature representing the diameter of the nodule.
The $v_r$ and $v_m$ are combined for at least one crop, but more preferably for every crop to form a feature vector with two features: risk of malignancy and diameter. The feature vectors for each patient are concatenated (670) and passed to the aggregation model (520). In this example, the aggregation model
(520) is configured to perform the following steps:
Discard feature vectors with measured size that is below a pre-defined size threshold (685). Select feature vectors with the highest risk of malignancy (690).
The output of the aggregation model is an aggregated feature vector $v_a$ for each patient containing the risk of malignancy and diameter of the nodule, above the configured size threshold, and with the highest risk of malignancy. The output of the aggregation model, and hence the feature extraction circuit, is a structured patient dataset containing these features for each patient (695).

**Distribution Circuit**

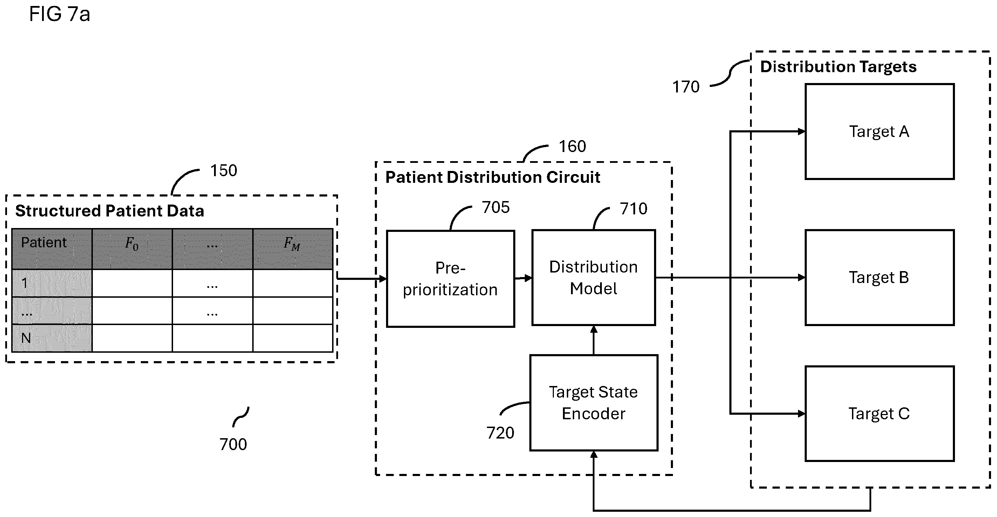

The patient distribution circuit (160) constitutes the third and final core component of the invention, and its task is to create a patient pre-prioritization list and distribution suggestion. Preferably, the patient distribution circuit comprises a distribution model that receives real time information from the structured patient database and the target state, and a target state encoder that also provides input

to the distribution model, wherein the distribution model produces an output of one or more distribution targets, to distribute patients according to patient and distribution target requirements. The general configuration (700) for the distribution circuit (160) is shown in FIGURE 7a. The distribution circuit takes as input the structured patient data (150), which contains the aggregated feature vectors $v_a$ output by the feature extraction circuit (140). The aggregated data is first organized according to a pre-prioritization rule (705) and then passed to the distribution model (710), which is configured to distribute the patients to a set of $N_T$ distribution targets (170). The pre-prioritization (705) allows the system to have a default behaviour for patient distribution and it is typically based on rules such as sorting by risk of disease, disease severity, condition urgency, patient arrival date/waiting time, etc. The state of the distribution targets is encoded by the target state encoder (720) and passed to the distribution model in addition to the structured patient data (150).

The target state encoder (720) projects the state of the $N_t$ distribution targets to a target state matrix $X_T \in \mathbb{R}^{N_t \times d_t}$, where $d_t$ is the number of features extracted from each target. The features $d_t$ characterize a target with information useful to deciding whether a given patient should be assigned to a given target. For example, type of clinic (e.g. urgent or non-urgent), expertise, and current capacity. In some examples of the distribution circuit (160), the distribution model (710) produces the distribution of patients to targets according to a static model, for example, a function of $v_a$ and static characteristics of the targets such as the clinical pathway.

A static distribution model is suitable for tasks such as assigning a patient to appropriate follow-up based on well-defined management guidelines such as Lung-RADS in lung cancer screening and BI-RADS in breast cancer screening, where positive-screen patients may be sent to a specialist, while negative-screen patients remain in the standard screening schedule.

In another example of the distribution circuit (160), the distribution of patients to targets is done according to a dynamic function, for instance, as a function of $v_a$ and $X_T$, where $X_T$ can change as a function of time. A dynamic example of the distribution model (710) is suitable when variables such as target capacity are important, for example in clinics with several specialists with varying expertise and working capacity. In a dynamic distribution model, the target state matrix $X_T$ is updated periodically, for example, every day, to reflect changes in the various variables characterizing the distribution targets such as target capacity. In a typical example of a dynamic distribution model, the assignment between patients and distribution targets is represented as an optimisation problem where each patient $p$ is assigned to each target $t$ incurring a cost $c_{pt}$. The cost $c_{pt}$ is a function of the features in the patient vector $v_a$ and the row of $X_T$ corresponding to the target, $v_t$. That is, $c_{pt} = Cost(v_a, v_t)$, where the function is designed such that a low cost $c_{pt}$ is indicative of a good fit between the patient and its assigned target (e.g. a patient with high risk of disease is sent to a target clinic dealing with urgent cases). Furthermore, each target can have a capacity variable $r_t$ which is accounted for during the assignment optimisation. In some examples of the dynamic distribution model, the goal of the optimisation problem is then to assign patients to distribution targets such that the sum of all assignment costs $c_{pt}$ is minimized. That is,

$$argmin_x \sum_{t=1}^{N_T} \sum_{p=1}^{N_p} c_{pt} . x_{pt} \qquad (2)$$

Here, $x_{pt}$ is an assignment variable indicating whether patient $p$ has been assigned to target $t$, and $N_p$ and $N_T$ are the number of patients and targets, respectively. The optimisation problem is solved in a distribution optimiser (920) step of the distribution model (710).

In some example implementations of the distribution circuit (160), the optimisation of assignments between patients and targets is done using the following method based on the Hungarian algorithm. The Hungarian algorithm is an optimisation algorithm designed to minimize the total cost of assigning a set of tasks to a set of agents. The algorithm operates under the constraints that each task is assigned to exactly one agent, and each agent is assigned to exactly one task. For the problem of distributing patients to targets, this means that the number of patients $N_p$ must match the number of targets $N_T$. In order to meet these requirements, if $N_p > N_T$, the patients are sequentially processed in batches of size $N_T$. In each batch, the Hungarian algorithm determines the optimal target assignment, and the distribution of patients is thus proposed accordingly. After each batch is processed, the capacity of each target $r_t$ is updated to account for the allocation of the previous batch. For cases where $N_P$ is not a multiple of $N_T$, or $N_p < N_T$, the final batch will not meet the required constraint that $N_p = N_T$. For these batches, a modification of the Hungarian algorithm which adds mock patients with a cost of zero for every target.

In another example of the distribution circuit (160), the optimisation of assignments between patients and targets is done as Linear Programming.

$$argmin_x \sum_{t=1}^{N_T} \sum_{p=1}^{N_p} c_{pt}.x_{pt} \qquad (3)$$

$$subject\ to$$

$$x_{pt} \in \{0, 1\}$$

$$\sum_{t=1}^{N_T} x_{pt} = 1 \ \forall \ p \in \{1, \dots, N_P\}$$

$$\sum_{p=1}^{N_P} x_{pt} \le r_t \ \forall \ t \in \{1, \dots, N_T\}$$

That is, the cost of assignment between patients and targets is minimized subject to each patient being assigned to exactly one target, and each target not exceeding its capacity.

In another example of the distribution circuit (160), if the patient assignment cannot be done without exceeding the capacity of the targets, the capacity of the targets is increased, and the patients enter a queue. In a different example, the list of patients is reduced according to the patient pre-prioritization (705), and the patient to target assignment problem is solved again with a reduced set of patients.

In some examples of the patient distribution circuit (160), the distribution model (710) distributes the patients following the order in the pre-prioritization (705).

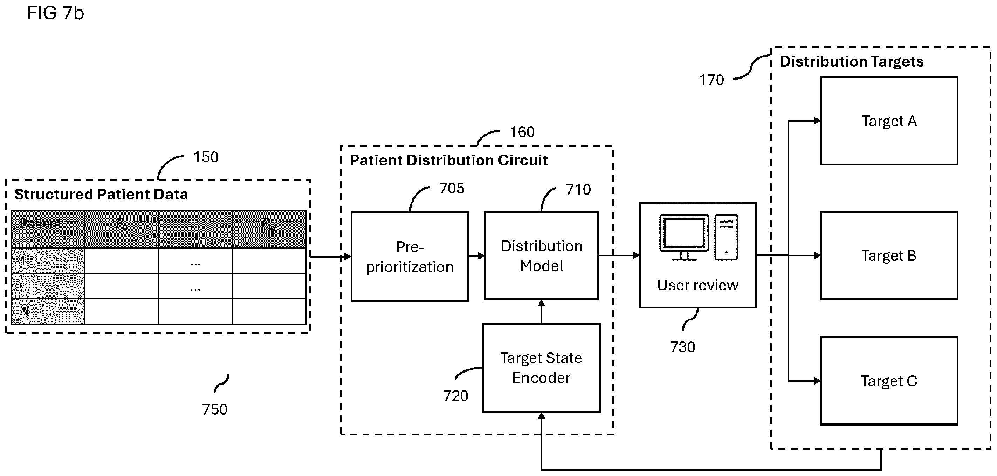

In some examples of the patient distribution circuit (160) with user intervention (750), depicted in FIGURE 7b, its output is presented to a system user (730) to review and edit the distribution proposal before the corresponding referrals are created. Preferably, at least one of the patient prioritisation list and the patient distribution list can be reviewed and/or edited by a user of the system.

Various embodiments of the patient distribution circuit (160) are described below.

*Distribution of patients with detected pulmonary nodules to appropriate follow- up*

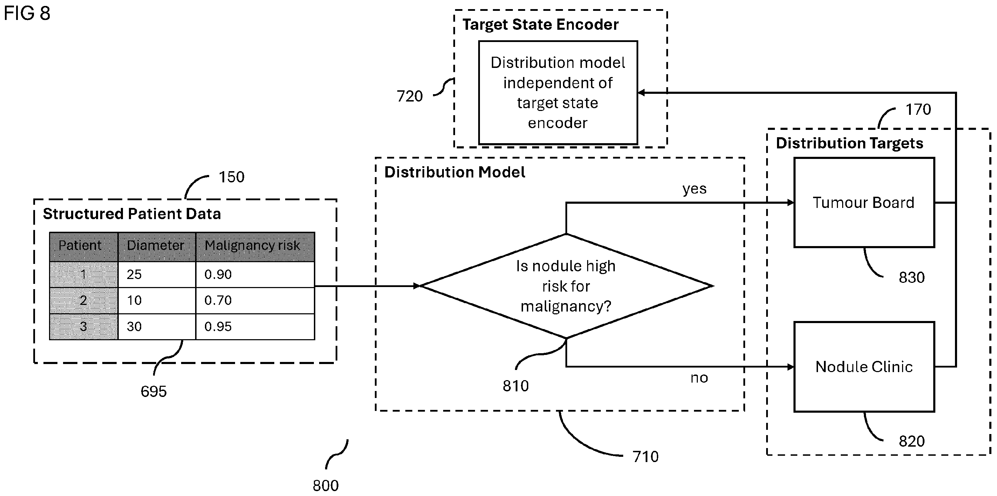

The configuration for this embodiment (800) is depicted in FIGURE 8. In this embodiment, there are two distribution targets: a nodule clinic for patients with low- and intermediate- risk pulmonary nodules (820), and a tumour board for patients with high-risk nodules (830). The distribution model (810) distributes patients with a risk of malignancy, for example, based on a threshold of the malignancy risk estimate or a size threshold, to the tumour board (830), with all other nodules distributed to the nodule clinic (820). This embodiment is an example of a static distribution model, given the state of the distribution targets has zero influence on the distribution model.

*Distribution of patients to clinicians of varying expertise and capacity*

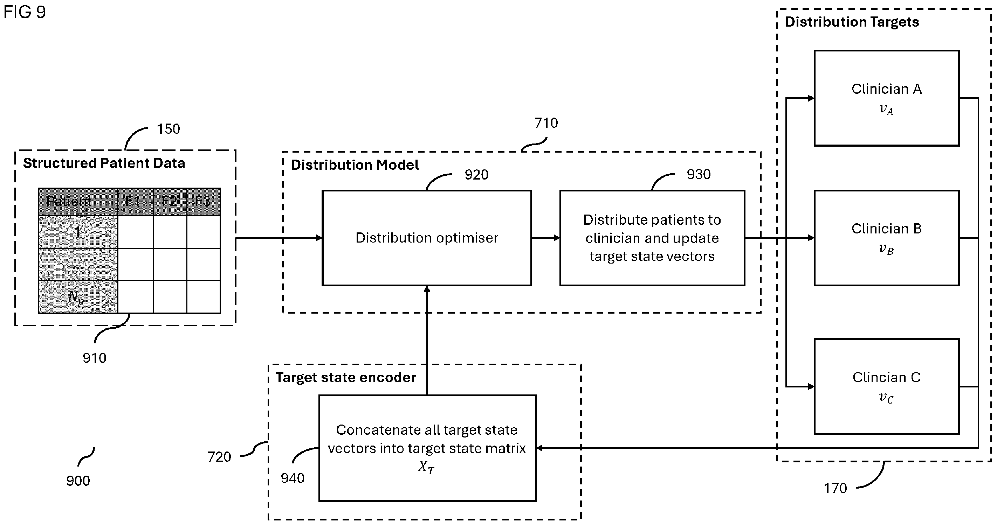

The general configuration for this embodiment (900) is depicted in FIGURE 9. In this embodiment, the distribution targets (170) are clinicians (clinician A, clinician B, clinician C). Each of the clinicians is described by a row in the target matrix $X_T$, which contains two columns (features):

Capacity: The current capacity of the clinician (0 = no capacity; 1 = completely available)
Expertise: The expertise of the clinician (0 = newly qualified; 1 = consultant)

Each patient is also described by a vector $v_a$ containing three features (910):

F1: Estimated condition severity .
F2: Patient age.
F3: The amount of time that the patient has been waiting for a referral.

In this example, the patient data (910) has been sorted according to the estimated condition severity in the pre-prioritization (705) of the patient distribution circuit (160).

The distribution model is configured to take as input $v_a$ and $X_T$, where $X_T$ is the concatenation of target state vectors (940) as produced by the target encoder (720). The assignment problem is then solved in the distribution model (710) by the distribution optimiser (920), after which the distribution is performed, and the target state vectors are updates accordingly (930).

**Embodiment of the invention for lung nodule patient discovery, pre-prioritization, and distribution**

The following is an example of how the three circuits are combined to form the patient workflow optimisation system for discovering, pre-prioritizing, and distributing patients with lung nodules for referral to one of several care pathways.

In this embodiment, the patient discovery circuit (120) searches for patients with a radiology report which contains keyword(s) indicating the presence of a lung nodule in at least one narrative radiology report. Patients with a radiology report indicating presence of a lung nodule are retrieved from the healthcare provider database (110). The data associated with the retrieved patients (130) are propagated to the feature extraction circuit (140).

The feature extraction circuit (140) takes as input the data retrieved (130) for the patients for the patients found by the discovery circuit (120), and processes, for each patient, the CT scan associated with the radiological report where the lung nodule was reported Specifically, the feature extraction model (510) applies three operations: (i) nodule detection : takes as input the whole CT image and outputs the 3D location, diameter and detection confidence (between 0 and 1) for each candidate above a defined detection confidence threshold; (ii) malignancy risk estimation: takes as input a CT volume centred at each nodule detection from (i), and produces outputs a malignancy risk score indicative of the likelihood that the lung nodule is malignant; (iii) nodule measurement: takes as input the same as (ii), segments the region of the CT volume which contains each nodule, and outputs its maximum axial diameter, volume and solid-to-subsolid volume ratio. The output of the feature extraction model (510) is a list of nodule detections for each patient with a concatenation of features derived from each component of the feature extraction model. Namely, maximum axial diameter, volume, solid-to-subsolid volume ratio, detection confidence and malignancy risk score.

For a given patient, the aggregation model (520) selects the actionable nodule with the highest risk of malignancy and passes it to the distribution circuit (160). The pre-prioritization (705) sorts the list of patients according to the malignancy risk score such that the patients with nodules most suspicious of lung cancer appear first.

Finally, the patient distribution model (710) takes as input the sorted list of patients according to the risk of malignancy of each patient. The distribution targets (170) are a set of clinical pathways: (i) refer to a nodule clinic for close monitoring, and (ii) refer to tumour board for further investigation. In an example of this embodiment, a decision threshold is configured by the user such that, any patient with a risk of malignancy score greater than the malignancy score threshold is referred to the tumour board, otherwise they are referred to a nodule clinic for monitoring. In a further example of this embodiment, the sorted list of patients according to the malignancy risk of their nodules, as well as the distribution schedule, is reviewed by a user of the system who decides to approve, reject, or edit the patient distribution schedule.

The system and method as described above has several technical advantages. In an embodiment, the system allows for the automation of a patient referral workflow which automatically finds, pre-prioritizes and suggests a distribution of patients to an appropriate care pathway, based on the raw data of the patients in a database within a healthcare system.

In an embodiment of the invention, the system may parse raw data from different modalities from a healthcare provider database, where the modalities include imaging data, structured clinical data, and unstructured clinical data. Preferably, the patient discovery circuit transforms raw clinical data to determine the subset of patients whose data match a search criterium.

In a preferred embodiment, the retrieved patient data subset is further processed by a feature extraction circuit to produce structured data from the raw patient data. Preferably, the feature extraction circuit aggregates the structured data of the retrieved patients to a single vector summarizing the patient features.

In an embodiment, a patient distribution circuit uses the summary of patient features to propose a patient pre-prioritization list and suggest a distribution of patients to one of several distribution targets.

Preferably, the current state of the distribution targets is accounted for the outcome of the patient distribution circuit.

In a preferred embodiment, the suggested distribution of patients can be reviewed and edited by a user of the system.

An example embodiment also provides a patient workflow system which enables the retrieval of relevant patient data from a healthcare database using natural language queries. In some examples, the patient queries are encoded by a query encoder to produce a query vector. Further preferably, the query vectors are combined with a patient vector to produce a patient relevancy for the query vector. Preferably, the patient relevancy for the query is used to retrieve the appropriate patients conditioned on the query.

In an example there is also provided a patient distribution model which combines patient features and distribution target features to suggest a patient distribution based on an optimization algorithm.

Some example advantages and benefits of the above-described system and method include:

End to end automation of patient allocation.

Retrieval of data (discovery circuit) -> extraction of features (feature extraction circuit) -> pre-prioritization and allocation to appropriate care pathway (discovery circuit).

System configuration for different tasks e.g. trial curation, clinic management.

The system may be configured multiple times for the same provider for different discovery circuit (for example different disease pathway)

Allows for configuration which facilitates natural language querying from a user to retrieve subjects which align with the particular query (discovery circuit).

Can be adaptable to the current capacity of the healthcare provider, ensuring patients needing the most are prioritised.

The system and method as described can be implemented in: Healthcare systems; Clinical trials; Clinical research organizations as well as other organizations that require the extraction of clinical data from hospital systems and networks.

The present invention has been described with reference to the accompanying drawings. However, it will be appreciated that the present invention is not limited to the specific examples herein described and as illustrated in the accompanying drawings. Furthermore, because the illustrated embodiments of the present invention may for the most part, be implemented using electronic components and circuits known to those skilled in the art, details will not be explained in any greater extent than that considered necessary as illustrated above, for the understanding and appreciation of the underlying concepts of the present invention and in order not to obfuscate or distract from the teachings of the present invention.

The invention may be implemented in a computer program for running on a computer system, at least including code portions for performing steps of a method according to the invention when run on a programmable apparatus, such as a computer system or enabling a programmable apparatus to perform functions of a device or system according to the invention.

A computer program is a list of instructions such as a particular application program and/or an operating system. The computer program may for instance include one or more of: a subroutine, a function, a procedure, an object method, an object implementation, an executable application, an applet, a servlet, a source code, an object code, a shared library/dynamic load library and/or other sequence of instructions designed for execution on a computer system. Therefore, some examples describe a non-transitory computer program product having executable program code stored therein for automated contouring of cone-beam CT images.

A computer process typically includes an executing (running) program or portion of a program, current program values and state information, and the resources used by the operating system to manage the execution of the process. An operating system (OS) is the software that manages the sharing of the resources of a computer and provides programmers with an interface used to access those resources. An operating system processes system data and user input, and responds by allocating and managing tasks and internal system resources as a service to users and programs of the system.

The computer system may for instance include at least one processing unit, associated memory and a number of input/output (I/O) devices. When executing the computer program, the computer system processes information according to the computer program and produces resultant output information via I/O devices.

In the foregoing specification, the invention has been described with reference to specific examples of embodiments of the invention. It will, however, be evident that various modifications and changes may be made therein without departing from the scope of the invention as set forth in the appended claims and that the claims are not limited to the specific examples described above.

Those skilled in the art will recognize that the boundaries between logic blocks are merely illustrative and that alternative embodiments may merge logic blocks or circuit elements or impose an alternate decomposition of functionality upon various logic blocks or circuit elements. Thus, it is to be understood that the architectures depicted herein are merely exemplary, and that in fact many other architectures can be implemented which achieve the same functionality.

Any arrangement of components to achieve the same functionality is effectively 'associated' such that the desired functionality is achieved. Hence, any two components herein combined to achieve a particular functionality can be seen as 'associated with' each other such that the desired functionality is achieved, irrespective of architectures or intermediary components. Likewise, any two components so associated can also be viewed as being 'operably connected,' or 'operably coupled,' to each other to achieve the desired functionality.

Furthermore, those skilled in the art will recognize that boundaries between the above-described operations merely illustrative. The multiple operations may be combined into a single operation, a single operation may be distributed in additional operations and operations may be executed at least partially overlapping in time. Moreover, alternative embodiments may include multiple instances of a particular operation, and the order of operations may be altered in various other embodiments.

However, other modifications, variations and alternatives are also possible. The specifications and drawings are, accordingly, to be regarded in an illustrative rather than in a restrictive sense.

In the claims, any reference signs placed between parentheses shall not be construed as limiting the claim. The word 'comprising' does not exclude the presence of other elements or steps then those listed in a claim. Furthermore, the terms 'a' or 'an,' as used herein, are defined as one or more than one. Also, the use of introductory phrases such as 'at least one' and 'one or more' in the claims should not be construed to imply that the introduction of another claim element by the indefinite articles 'a' or 'an' limits any particular claim containing such introduced claim element to inventions containing only one such element, even when the same claim includes the introductory phrases 'one or more' or 'at least one' and indefinite articles such as 'a' or 'an.' The same holds true for the use of definite articles. Unless stated otherwise, terms such as 'first' and 'second' are used to arbitrarily distinguish between the elements such terms describe. Thus, these terms are not necessarily intended to indicate temporal or other prioritization of such elements. The mere fact that certain measures are recited in mutually different claims does not indicate that a combination of these measures cannot be used to advantage.

## Claims

1. A workflow optimisation system to perform at least one of:

    prioritisation of patients for a specific treatment pathway;
    distribution of patients to a specified treatment pathway;

    the system comprising:

    a database comprising medical data for one or more patients;
    a patient discovery circuit for receiving medical data for one or more patients from the database to transform the received medical data to retrieved patient data;
    a feature extraction circuit configured to process the retrieved patient data to produce structured data for each of the one or more patients, and aggregate the structured data to a single vector for each patient, where the single vector summarises medical features for the patient; and
    a patient distribution circuit for receiving the single vector for each patient and determining, from the single vector for each patient, at least one of: a patient prioritisation list to prioritise patients for distribution, and a patient distribution list to distribute the patients to one or more distribution targets.

2. A workflow optimisation system as in claim 1, where in the medical data comprises at least one of: medical imaging data, structured data, unstructured data.

3. A workflow optimisation system as claimed in claim 1 or claim 2, wherein the patient prioritisation list and the patient distribution list can be reviewed and/or edited by a user of the system.

4. A workflow optimisation system as in any preceding claim, wherein the medical data is retrieved from the database in response to one of more natural language queries.

5. A workflow optimisation system as in any preceding claim, wherein the patient discovery circuit comprises a data transformation model to transform the received medical data, wherein the data is transformed by one or more of: -restructuring or reformatting the data, removing patient identifiable information, removing any other unnecessary data, producing data summaries, encoding raw data such medical images to facilitate their parsing, and a patient retrieval model, wherein the patient retrieval model analyses the transformed data., wherein the patient retrieval model analyses the transformed data to determine the presence of at least one of: a predefined regular expression string (regex); a match to a predefined database query.

6. A workflow optimisation system as in claim 5, wherein the data transformation model comprises an encoder for each of the medical imaging data, the structured data and the unstructured data, and each encoder outputs a vector for each of the medical imaging data, the structured data and the unstructured data, wherein the output vectors are input to a embedding merger to be fused to generate the single output vector for a patient, and wherein the output vectors for all patients are concatenated to generate a patient matrix, $X_p$.

7. A workflow optimisation system as in claim 6, wherein the patient matrix $X_p$, is provided as an input to the data retrieval model, and a natural language query is input to a query encoder in the data retrieval model, and an output vector from the query encoder is provided to the data retrieval model, and is combined with the patient matrix to generate a relevance score, $r_q$, for each patient, which indicates how relevant is each patient in $X_p$ to the natural language query.

8. A workflow optimisation system as in claim 7, wherein the relevance score is generated using a similarity metric between the patient features and the encoded natural language query, and wherein the retrieval model is configured to select one or more patients based on a comparison of the relevance score with a predetermined threshold.

9. A workflow optimisation system as in claim 7, wherein one or more of the encoders is a neural network.

10. A workflow optimisation system as claimed in any preceding claim, wherein the feature extraction circuit comprises a feature extraction model to extract one or more feature vectors from the retrieved patient data and an aggregation model, that receives the one or more feature vectors, and produces an output of one aggregated vector per patient, wherein the aggregated vectors for each patient are concatenated to produce a structured patient database.

11. A workflow optimisation system as claimed in any preceding claim, wherein the feature extraction model comprises the detection and characterization of a medical entity in the medical data, and outputs a feature vector comprising the entity location, detection confidence parameter, and entity characterization information for medical imaging data from one or more patients., wherein the medical entity is a nodule, and the feature vector can be used to determine one or more of nodule malignancy risk, nodule size, nodule attenuation, and other clinical parameters for the nodule for one or more of the patients.

12. A workflow optimisation system as claimed in any preceding claim, wherein the patient distribution circuit comprises a distribution model that receives information from the structured patient database and a target state encoder that also provides real-time information about the distribution targets as input to the distribution model, wherein the distribution model produces an output indicating a distribution of patients to distribution targets according to patient and distribution target requirements.

13. A workflow optimisation system as in claim 12, wherein the distribution model is a static model, where the state of the distribution targets is fixed in time.

14. A workflow optimisation system as in claim 12, wherein the distribution model is a dynamic model, where the target state encoder generates a target state matrix, $X_T$, where $X_T$ will vary as a function of time.

15. A workflow optimization method for the prioritisation and distribution of patients to a specified treatment pathway comprising:

> receiving and storing medical data for one or more patients in a patient database;
> receiving medical data for one or more patients from the database at a patient discovery circuit to transform the received medical data to retrieved patient data;
> processing the retrieved patient data in a feature extraction circuit to produce structured data for each of the one of more patients, and aggregating the structured data to a single vector for each patient, where the single vector summarises medical features for the patient; and
> receiving the single vector for each patient at a patient distribution circuit and determining at least one of: a patient prioritisation list and a patient distribution list to prioritize and distribute the patients to one or more distribution targets.

FIG 1

EP 4 657 453 A1

FIG 2

**FIG 3**

Healthcare Provider Database — 110, 115

Per-patient dataset
- Medical image(s) — 111
- Structured data — 112
- Unstructured data — 113

Data transformation model — 220
- Get radiology reports — 310
- De-identify PHI in report — 320
- Extract relevant sections from report — 330

Retrieval model — 230, 300

Data retriever — 232
- Retrieve match list

Patient generator — 231
- Generate list of patients which match regex

Config = r"(?=.*(lung|pulmonary)(?=.*(nodule|mass)).+" — 340

Retrieved patient data — 130

EP 4 657 453 A1

EP 4 657 453 A1

FIG 4a

FIG 4b

FIG 5

FIG 6a

Figure 6a — Feature Extraction Model flow diagram (130 Retrieved Patient Data, 510 Feature Extraction Model)

130 — Retrieved Patient Data
610 — Latest CT Image
600
510 — Feature Extraction Model
620 — Nodule detection
630 — (table: Patient | Candidate | x | y | z | c)
640 — Crop CT volume around candidate x, y, z if c>threshold
650 — Nodule malignancy risk
660 — Nodule measurement
670 — (results table)

| Patient | Candidate | x | y | z | c |
|---------|-----------|---|---|---|---|
| 1 | 1 | | | | |
| 1 | 2 | | | | |
| 1 | 3 | | | | |
| 2 | 1 | | | | |
| 2 | 2 | | | | |
| 3 | 1 | | | | |
| 3 | 2 | | | | |
| 3 | 3 | | | | |

| Patient | Candidate | Diameter | Malignancy risk |
|---------|-----------|----------|-----------------|
| 1 | 1 | 5 | 0.1 |
| 1 | 2 | 25 | 0.9 |
| 1 | 3 | 15 | 0.3 |
| 2 | 1 | 10 | 0.8 |
| 2 | 2 | 6 | 0.7 |
| 3 | 1 | 30 | 0.9 |
| 3 | 2 | 4 | 0.4 |
| 3 | 3 | 9 | 0.6 |

FIG 6b

| Patient | Candidate | Diameter | Malignancy risk |
|---------|-----------|----------|-----------------|
| 1 | 1 | 5 | 0.10 |
| | 2 | 25 | 0.90 |
| | 3 | 15 | 0.30 |
| 2 | 1 | 10 | 0.70 |
| | 2 | 6 | 0.40 |
| 3 | 1 | 30 | 0.95 |
| | 2 | 4 | 0.40 |
| | 3 | 9 | 0.60 |

670

⌒ 520

**Aggregation Model**

Discard
candidates
below size
threshold

Select the
candidate with
highest
malignancy risk

685    690

680

⌒ 150

**Structured Patient Data**

| Patient | Diameter | Malignancy risk |
|---------|----------|-----------------|
| 1 | 25 | 0.90 |
| 2 | 10 | 0.70 |
| 3 | 30 | 0.95 |

695

FIG 7a

FIG 7b

**Distribution Targets** 170

Target A

Target B

Target C

730 User review

**Patient Distribution Circuit** 160

705 Pre-prioritization

710 Distribution Model

720 Target State Encoder

**Structured Patient Data** 150

| Patient | $F_0$ | ... | $F_M$ |
|---|---|---|---|
| 1 | | | |
| ... | ... | ... | |
| N | | | |

750

FIG 8

**Target State Encoder**

Distribution model independent of target state encoder

720

170

**Distribution Targets**

**Distribution Model**

150

**Structured Patient Data**

| Patient | Diameter | Malignancy risk |
|---|---|---|
| 1 | 25 | 0.90 |
| 2 | 10 | 0.70 |
| 3 | 30 | 0.95 |

695

yes

Tumour Board

830

Is nodule high risk for malignancy?

810

no

Nodule Clinic

820

710

800

FIG 9

EP 4 657 453 A1

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2024/100632 A1 (JOHNSON & JOHNSON ENTPR INNOVATION INC [US]) 16 May 2024 (2024-05-16) * abstract * * paragraphs [0004], [0040], [0145], [0146] * ----- | 1-15 | INV. G16H40/20 |
| A | KARUNAKARAN BIPIN ET AL: "Closing the loop - Finding lung cancer patients using NLP", 2017 IEEE INTERNATIONAL CONFERENCE ON BIG DATA (BIG DATA), IEEE, 11 December 2017 (2017-12-11), pages 2452-2461, XP033298516, DOI: 10.1109/BIGDATA.2017.8258203 [retrieved on 2018-01-12] * abstract * ----- | 1-15 | |

**TECHNICAL FIELDS SEARCHED (IPC)**

G16H

The present search report has been drawn up for all claims

| Place of search | Date of completion of the search | Examiner |
|---|---|---|
| Munich | 29 September 2025 | Schmitt, Constanze |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
    ..............................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 25 17 8439

This annex lists the patent family members relating to the patent documents cited in the above-mentioned European search report.
The members are as contained in the European Patent Office EDP file on
The European Patent Office is in no way liable for these particulars which are merely given for the purpose of information.

29-09-2025

| Patent document cited in search report | Publication date | Patent family member(s) | Publication date |
|---|---|---|---|
| WO 2024100632 A1 | 16-05-2024 | NONE | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LUMBRERAS, B.** ; **DONAT, L.** ; **HERNA, I.** ; **HERNANDEZ, M.** *Incidental findings in imaging diagnostic tests: a systematic review.*, https://doi.org/10.1259/bjr/98067945

- **RÖHRICH, S.** ; **HEIDINGER, B. H.** ; **PRAYER, F.** ; **WEBER, M.** ; **KRENN, M.** ; **ZHANG, R.** ; **SUFANA, J** ; **SCHEITHE, J.** ; **KANBUR, I.** ; **KORAJAC, A.** *Impact of a content-based image retrieval system on the interpretation of chest CTs of patients with diffuse parenchymal lung disease*, https://doi.org/10.1007/s00330-022-08973-3/Published